(19) Europäisches Patentamt
European Patent Office
Office européen des brevets

(11) Numéro de publication: **0 062 551**
**A1**

(12) # DEMANDE DE BREVET EUROPEEN

(21) Numéro de dépôt: **82400411.3**

(22) Date de dépôt: **09.03.82**

(51) Int. Cl.³: **C 07 D 235/18**

(30) Priorité: **20.03.81 FR 8105587**

(43) Date de publication de la demande: **13.10.82**
**Bulletin 82/41**

(84) Etats contractants désignés: **BE CH DE FR GB IT LI NL**

(71) Demandeur: **P C U K PRODUITS CHIMIQUES UGINE KUHLMANN, Service Propriété Industrielle Tour Manhattan, F-92087 Paris La Defense 2 Cédex 21 (FR)**

(72) Inventeur: **Cognion, Jean-Marie, 85, route de Charly, F-69230 Saint Genis Laval (FR)**
Inventeur: **Durual, Pierre, Les Essarts No. 2 Chemin du Rossignol, F-69390 Vernaison (FR)**

(74) Mandataire: **Leboulenger, Jean et al, P C U K PRODUITS CHIMIQUES UGINE KUHLMANN Service Propriéte Industrielle Tour Manhattan, F-92087 Paris La Défense 2 Cedex 21 (FR)**

(54) **Procédé de préparation de l'(amino-4 phényl)-2 amino-5 benzimidazole et de dérivés substitués.**

(57) L'invention concerne un procédé de préparation de 1'-(amino-4-phényl)-2-amino-5-benzimidazole et de dérivés substitués, par réduction catalytique de la N-(nitro-4-benzoyl)-dinitro-2,4-aniline (TNB) ou d'un dérivé substitué par l'hydrogène gazeux.

Selon l'invention, le TNB ou son dérivé substitué est traité en suspension dans une solution aqueuse d'acide chlorhydrique ou phosphorique, la teneur (C) de cette suspension en TNB ou dérivé substitué allant de 0,15 à 1,5 mole par litre et le rapport molaire (A): acide/TNB ou dérivé substitué allant de 2 à 20. On opère en outre sous une pression d'hydrogène allant de 5 à 100 bars et à une température (T), comprise entre 20 et 150 °C, telle que le produit T × A × C soit au moins égal à 25.

On obtient ainsi un produit d'excellente pureté avec des rendements pratiquement quantitatifs.

EP 0 062 551 A1

Procédé de préparation de l'(amino-4 phényl)-2 amino-5 benzimidazole et de dérivés substitués

La présente invention concerne un nouveau procédé de préparation de l'(amino-4 phényl)-2 amino-5 benzimidazole (en abrégé DAPBI) et de dérivés substitués.

On connait diverses voies d'accès au DAPBI ou à ses dérivés substitués par des atomes d'halogène ou des groupes alkyle ou alcoxy sur les noyaux aromatiques.

Dans les brevets français 2 297 849 et 2 332 273, on a proposé une synthèse en deux étapes à partir de la N(nitro-4 benzoyl) dinitro-2,4 aniline, consistant en (a) une réduction chimique au moyen de sulfhydrate, de sulfure ou de polysulfure de sodium ou d'ammonium en $N_1$-(amino-4 benzoyl) triamino-1,2,4 benzène, puis (b) une cyclodéshydratation de cette triamine en milieu acide conduisant au DAPBI ou à un de ses chlorhydrates. Cette technique ne permet malheureusement pas d'obtenir des rendements molaires en DAPBI par rapport au composé trinitro engagé, supérieurs à 80 %. En effet, le milieu réducteur fortement basique provoque également des réactions secondaires d'hydrolyse qui diminuent à la fois le rendement et la qualité du produit recherché ; on constate la formation d'acide p-aminobenzoïque en quantité non négligeable. En outre, cette technique de réduction chimique sous-produit des quantités très importantes d'effluents pollués par des composés soufrés dont l'épuration avant rejet est coûteuse.

Dans la revue Zhur. Obshchei Khim 28, 928, 1958 (Chem. Abst. 52, 17240, 1958), B.A. PORAI-KOSHITS et Ch. FRANKOVSKII ont proposé également une cycloréduction chimique de la N-(nitro-4 benzoyl) dinitro-2,4 aniline par le couple $SnCl_2$, HCl. Cette technique présente cependant de sérieux inconvénients, à savoir :

- un coût élevé de l'opération car le chlorure stanneux n'est pas un produit industriel courant ;

- une importante pollution par les sous-produits ;
- des rendements molaires en DAPBI limités à 74-75 % par rapport aux produits de départ : le chlorure de p-nitrobenzoyle et la dinitro-2,4 aniline.

Dans le brevet US 4 192 947, on a proposé une synthèse du DAPBI en une seule étape à partir de la $N_1$-(nitro-4 benzoyl)nitro-5 o-phénylènediamine, par cycloréduction en milieu fortement basique, en phase aqueuse ou dans un solvant organique. Le nitro-4 diamino-1,2 benzène, nécessaire pour préparer la $N_1$-(nitro-4 benzoyl) nitro-5 o-phénylènediamine par condensation avec le chlorure de p-nitrobenzoyle, est une matière première coûteuse. D'autre part, les rendements molaires annoncés en DAPBI par rapport au nitro-diamino-benzène sont de l'ordre de 84 % avec une pureté de 91-92 %.

La réduction catalytique de la N-(nitro-4 benzoyl) dinitro-2,4 aniline par l'hydrogène gazeux est connue (brevet U.R.S.S. 546 608 résumé dans Chem. Abst. 87, 22808t, 1977), mais aucune réalisation industrielle n'en a été envisagée. Elle nécessite l'emploi, en quantité importante, de solvants coûteux comme le N,N-diméthylformamide, la N-méthyl-pyrrolidone, l'aniline ou le dioxanne et ne permet pas d'accéder au DAPBI en une seule étape ; on obtient, avec des rendements souvent médiocres, la triamine correspondante, parfois cristallisée avec du solvant. C'est aussi cette triamine qu'on obtient lorsqu'on effectue la réduction en milieu acide très dilué dans les conditions décrites par SHCHEL'TSYN et al. dans la revue Sint. Anal. Strukt. Org. Soedin. 1972, No.4, 64-8).

Il a maintenant été trouvé qu'on peut obtenir en une seule étape et en l'absence de tout solvant organique un DAPBI d'excellente pureté avec des rendements pratiquement quantitatifs, lorsqu'on soumet la N-(nitro-4 benzoyl) dinitro-2,4 aniline en suspension dans une solution aqueuse d'acide chlorhydrique ou phosphorique à une cycloréduction catalytique par l'hydrogène gazeux,

selon le schéma réactionnel suivant :

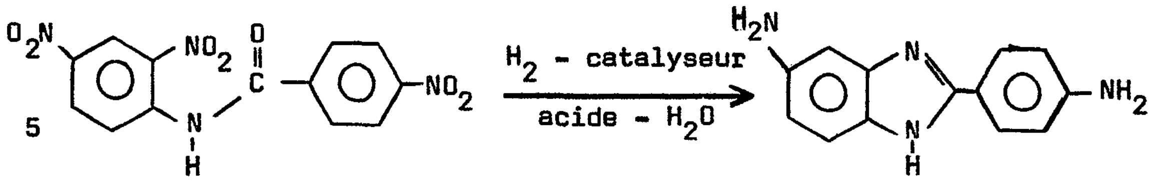


Les catalyseurs à employer dans le procédé selon l'invention sont les catalyseurs classiques d'hydrogénation des fonctions nitro-aromatiques en amines, c'est-à-dire les catalyseurs à base d'un métal du groupe VIII de la classification périodique des éléments, tel que le nickel, le platine, le palladium, le rhodium et le ruthénium. Ces métaux peuvent être déposés ou non sur des supports comme, par exemple, l'alumine, les silices alumines, la magnésie, le charbon actif. Des formules bimétalliques du type Pt-Pd sont également très intéressantes. La quantité de métal engagé peut atteindre 5 g par kg de N-(nitro-4 benzoyl) dinitro-2,4 aniline, mais on préfère une quantité comprise entre 25 et 500 mg/kg.

La teneur (C) de la suspension aqueuse acide en N-(nitro-4 benzoyl) dinitro-2,4 aniline peut aller de 0,15 à 1,5 mole par litre. Le rapport molaire (A) : acide/N-(nitro-4 benzoyl) dinitro-2,4 aniline peut aller de 2 à 20.

On opère sous une pression d'hydrogène allant de 5 à 100 bars, de préférence de 20 à 50 bars, et à une température (T), allant de 20 à 150°C, telle que le produit T x A x C soit au moins égal à 25. De préférence, on opère à une température comprise entre 20 et 100°C telle que le produit T x A x C soit au moins égal à 50.

Les vitesses d'hydrogénation sont élevées et le sel de DAPBI formé au cours de la réaction passe immédiatement en solution. Lorsque la réaction est terminée, une simple filtration à chaud permet la séparation et la récupération du catalyseur. Par refroidissement et éventuellement évaporation du filtrat, on peut récupérer le sel de DAPBI

sous forme cristallisée. Le DAPBI peut être isolé sous forme de base libre par toute méthode connue. Pour la fabrication de colorants azoïques, on peut aussi utiliser le filtrat tel quel.

La présente invention inclut également la préparation de dérivés substitués du DAPBI à partir des dérivés substitués correspondants de la N-(nitro-4 benzoyl) dinitro-2,4 aniline.

Les exemples suivants, dans lesquels les pourcentages indiqués sont en poids, illustrent l'invention, sans la limiter.

EXEMPLE 1

Dans un autoclave en Hastelloy C de 1 litre, muni d'un dispositif d'agitation rotatif magnétique et d'un dispositif de chauffage par circulation d'huile, on charge 20 g (0,06 mole) de N-(nitro-4 benzoyl) dinitro-2,4 aniline, 135 g d'eau, 45 g d'une solution d'HCl à 35,5 % (0,44 mole) et 0,2 g d'un catalyseur Pd/charbon actif contenant 50 % d'eau et 2,5 % de palladium. Après balayage à l'azote, on introduit l'hydrogène à la pression de 30 bars et chauffe jusqu'à 90°C. Dès que cette température est atteinte, la mise en service de l'agitation provoque une baisse de la pression d'hydrogène que l'on maintient à 30 bars par un appoint. Après 4 heures de réaction, la consommation d'hydrogène s'arrête et l'on récupère dans l'autoclave une solution chaude que l'on sépare immédiatement du catalyseur en suspension par filtration. Par refroidissement et évaporation, on récupère à partir de cette solution 20,6 g (0,06 mole) de trichlorhydrate de DAPBI identifié par spectrographie infra-rouge.

5 g (0,015 mole) de ce trichlorhydrate sont remis en solution aqueuse et neutralisés jusqu'à pH 11 par 50 ml d'une solution normale d'hydroxyde de sodium (0,05 mole). Après filtration, lavage à l'eau et recristallisation dans l'éthanol, on récupère 3,45 g (0,0143 mole) de DAPBI cristallisé avec une mole d'eau. Le spectre infra-rouge est en bon accord avec celui du produit obtenu par réduction chimique selon le brevet français 2 297 849. L'analyse

élémentaire donne des valeurs correspondant bien à la formule brute $C_{13}H_{12}N_4$, $H_2O$ de masse moléculaire 242 :

| | Calculé | Trouvé |
|---|---|---|
| % C | 64,46 | 64,44 |
| % H | 5,78 | 5,89 |
| % N | 23,14 | 22,84 |

La nature et la pureté du produit obtenu ont également été confirmées par RMN.

EXEMPLE 2

Dans l'autoclave décrit à l'exemple 1, on introduit 60 g (0,18 mole) de N-(nitro-4 benzoyl) dinitro-2,4 aniline, 405 g d'eau, 135 g d'une solution d'HCl à 35,5 % (1,3 mole) et 0,6 g du même catalyseur qu'à l'exemple 1. Après 5,5 heures de réaction dans les mêmes conditions qu'à l'exemple 1 (90°C et 30 bars), filtration du catalyseur, refroidissement et évaporation, on obtient 60,5 g (0,18 mole) de trichlorhydrate de DAPBI identifié par spectrographie infra-rouge.

EXEMPLE 3

Dans l'autoclave décrit à l'exemple 1, on introduit 60 g (0,18 mole) de N-(nitro-4 benzoyl) dinitro-2,4 aniline, 135 g d'eau, 70 g d'une solution d'HCl à 35,5 % (0,68 mole) et 0,6 g du même catalyseur qu'à l'exemple 1. Après 4 heures de réaction dans les mêmes conditions qu'à l'exemple 1 (90°C et 30 bars), filtration du catalyseur, refroidissement et évaporation, on obtient 60 g (0,18 mole) de trichlorhydrate de DAPBI identifié par spectrographie infra-rouge.

EXEMPLE 4

On opère comme à l'exemple 1, mais en remplaçant les 0,2 g de catalyseur au palladium par 0,1 g d'un catalyseur sec contenant 5 % de rhodium sur charbon actif (95 %). Après 3,5 heures de réaction, on récupère 20 g de trichlorhydrate de DAPBI identifié par spectrographie infra-rouge.

EXEMPLE 5

On opère comme à l'exemple 1, mais en remplaçant les 0,2 g de catalyseur au palladium par 0,1 g d'un catalyseur sec contenant 5 % de platine sur charbon actif (95 %). Après 2,25 heures de réaction on récupère 20 g de trichlorhydrate DAPBI identifié par spectrographie infra-rouge. 10 g (0,03 mole) de ce trichlorhydrate sont remis en solution aqueuse et neutralisés jusqu'à pH 10 par 90 ml d'une solution normale d'hydroxyde de sodium. Après filtration et lavage à l'eau, on récupère 7 g (0,029 mole) de DAPBI cristallisé avec une mole d'eau. Les spectres infra-rouge et R.M.N. de ce produit sont en bon accord avec les spectres de DAPBI obtenus précédemment.

EXEMPLE 6

On répète l'exemple 1 à trois températures différentes (90, 50 et 20°C) et en remplaçant les 0,2 g de catalyseur au palladium par 0,25 g d'un catalyseur bimétallique contenant 2 % de palladium et 0,2 % de platine sur charbon actif (97,8 %), ce catalyseur étant introduit dans le réacteur sous forme d'une suspension aqueuse contenant 35 g de solide par litre. Les résultats obtenus sont rassemblés dans le tableau suivant :

| T°C | Durée en heures | Trichlorhydrate de DAPBI | |
|---|---|---|---|
| | | g | mole |
| 90 | 0,75 | 20 | 0,06 |
| 50 | 2 | 18,10 | 0,055 |
| 20 | 2,5 | 20 | 0,06 |

EXEMPLE 7

Dans l'autoclave décrit à l'exemple 1, on charge 20 g (0,06 mole) de N-(nitro-4 benzoyl) dinitro-2,4 aniline, 135 g d'eau, 87 g d'acide phosphorique commercial à 85 % (0,76 mole) et 0,4 g du même catalyseur qu'à l'exemple 1.

Après 1,75 heure de réaction dans les mêmes conditions qu'à l'exemple 1 (90°C, 30 bars), filtration du catalyseur, lavage à l'eau et refroidissement, on obtient 238 ml d'une solution de phosphate de DAPBI que l'on dose par chromatographie en phase liquide sous pression selon la méthode "reverse-phase" par appariement d'ions, avec l'heptane-sulfonate de sodium comme contre-ion.

On trouve ainsi une teneur de 46,4 g/l de DAPBI, ce qui représente 0,0493 mole, soit un rendement de 82,2 %.

EXEMPLE 8

Dans l'autoclave décrit à l'exemple 1, on introduit 10 g (0,03 mole) de N-(nitro-4 benzoyl) dinitro-2,4 aniline, 130 g d'eau, 14 g d'acide phosphorique commercial à 85 % (0,12 mole) et 0,8 g du même catalyseur qu'à l'exemple 1. Après 15 minutes de réaction dans les mêmes conditions qu'à l'exemple 1 (90°C, 30 bars), filtration et lavage à l'eau du catalyseur puis refroidissement, on obtient 345 ml d'une solution de phosphate de DAPBI dont la teneur mesurée par chromatographie en phase liquide est de 15,8 g/l de DAPBI, ce qui représente 0,0243 mole, soit un rendement de 81 %.

EXEMPLE 9

Dans l'autoclave décrit à l'exemple 1, on charge 10 g (0,03 mole) de N-(nitro-4 benzoyl) dinitro-2,4 aniline, 150 g d'eau, 43,5 g d'acide phosphorique commercial à 85 % (0,38 mole) et 0,4 g du même catalyseur qu'à l'exemple 1. Après une heure de réaction dans les mêmes conditions qu'à l'exemple 1 (90°C, 30 bars), filtration du catalyseur et refroidissement, on obtient 186 ml d'une solution de phosphate de DAPBI dont la teneur mesurée par chromatographie en phase liquide est de 33,5 g/l de DAPBI, ce qui représente 0,0278 mole, soit un rendement de 92,7 %.

Revendications de brevet

1. Procédé de préparation de l'(amino-4 phényl)-2 amino-5 benzimidazole (en abrégé DAPBI) et de dérivés substitués, par réduction de la N-(nitro-4 benzoyl) dinitro-2,4 aniline (en abrégé TNB) ou d'un dérivé substitué au moyen d'hydrogène gazeux en présence d'un catalyseur à base d'un métal du groupe VIII de la classification périodique des éléments, caractérisé en ce que le TNB ou son dérivé substitué est en suspension dans une solution aqueuse d'acide chlorhydrique ou phosphorique, la teneur (C) de ladite suspension en TNB ou dérivé substitué allant de 0,15 à 1,5 mole par litre et le rapport molaire (A) : acide/TNB ou dérivé substitué allant de 2 à 20, et qu'on opère sous une pression d'hydrogène allant de 5 à 100 bars et à une température (T), comprise entre 20 et 150°C, telle que le produit T x A x C soit au moins égal à 25.

2. Procédé selon la revendication 1 dans lequel on utilise un catalyseur à base de nickel, de platine, de palladium, de rhodium et/ou de ruthénium.

3. Procédé selon la revendication 2 dans lequel on utilise un catalyseur à base de platine, de palladium ou de rhodium déposé sur un support.

4. Procédé selon la revendication 2 dans lequel on utilise un catalyseur à base de platine et de palladium déposés sur un support.

5. Procédé selon l'une des revendications 1 à 4 dans lequel on utilise 25 à 500 mg de métal par kg de TNB ou de dérivé substitué.

6. Procédé selon l'une des revendications 1 à 5 dans lequel on opère sous une pression d'hydrogène allant de 20 à 50 bars.

7. Procédé selon l'une des revendications 1 à 6 dans lequel on opère à une température (T), comprise entre 20 et 100°C, telle que le produit T x A x C est au moins égal à 50.

8. Procédé selon l'une des revendications 1 à 7 dans lequel on obtient une solution d'un chlorhydrate ou phosphate de DAPBI ou de dérivé substitué.

9. Procédé selon la revendication 8 dans lequel on isole le DAPBI ou son dérivé substitué sous forme de sel, de préférence sous forme de trichlorhydrate.

0062551

Office européen des brevets

# RAPPORT DE RECHERCHE EUROPEENNE

Numéro de la demande

EP 82 40 0411

## DOCUMENTS CONSIDERES COMME PERTINENTS

| Categorie | Citation du document avec indication, en cas de besoin, des parties pertinentes | Revendication concernée | CLASSEMENT DE LA DEMANDE (Int. Cl. 3) |
|---|---|---|---|
| D,A | EP - A - 0 001 246 (CASSELLA)<br>---- | | C 07 D 235/18 |

| DOMAINES TECHNIQUES RECHERCHES (Int. Cl. 3) |
|---|
| C 07 D 235/00 |

Le présent rapport de recherche a été établi pour toutes les revendications

| Lieu de la recherche | Date d'achèvement de la recherche | Examinateur |
|---|---|---|
| La Haye | 24-06-1982 | DE BUYSER |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

... rm 1503 03 82